# EUROPEAN PATENT SPECIFICATION

(11) **EP 1 847 235 B1**
(45) Date of publication and mention of the grant of the patent: **18.08.2010**
(21) Application number: 07007912.4
(22) Date of filing: 18.04.2007
(51) Int. Cl.: A61F 2/06

(54) **Devices for contributing to improved stent graft fixation**
Vorrichtungen, die zu einer verbesserten Stent-Graft-Befestigung beitragen
Dispositifs pour contribuer à une fixation améliorée de greffes d'endoprothèses

(30) Priority: 18.04.2006 US 379110
(43) Date of publication of application: 24.10.2007
(73) Proprietor: Medtronic Vascular, Inc., Santa Rosa, CA 95403 (US)
(72) Inventor: Schulman, Seth, Cambridge Massachusetts 02139 (US)
(74) Representative: Zimmermann, Gerd Heinrich

(56) References cited:
- EP-A- 1 543 798
- WO-A-2004/060424
- WO-A1-99/43379
- US-A- 5 342 348
- US-A1- 2001 053 931
- US-B1- 6 221 102
- BARNER S S: "ABSORBABLE IRIS SUTURE AN EXPERIMENTAL EVALUATION OF POLY GLYCOLIC-ACID SUTURE" 1978, ALBRECHT VON GRAEFES ARCHIV FUER KLINISCHE UND EXPERIMENTELLE OPHTHALMOLOGIE, SPRINGER, BERLIN, DE, PAGE(S) 29-38 , XP008082339 ISSN: 0065-6100 * page 35, lines 12-15 *

## Description

### FIELD OF THE INVENTION

The present invention relates to devices to contribute to improved stent graft fixation within vessels at treatment sites. More specifically, the present invention relates and devices to contribute to improved stent graft fixation within vessels at treatment sites by providing stent grafts and methods of making and using stent grafts with bare metal portions comprising a substance that promotes an inflammatory response.

### BACKGROUND OF THE INVENTION

Stent grafts have been developed to treat abnormalities of the vascular system. Stent grafts are primarily used to treat aneurysms of the vascular system and have also emerged as a treatment for a related condition, acute blunt aortic injury, where trauma causes damage to an artery.

Aneurysms arise when a thinning, weakening section of a vessel wall balloons out. Aortic aneurysms (both abdominal and thoracic) are treated when the vessel wall expands to more than 150% of its normal diameter. These thinned and weakened sections of vessel walls can burst, causing an estimated 32,000 deaths in the United States each year. Additionally, aneurysm deaths are suspected of being underreported because sudden unexplained deaths, about 450,000 in the United States alone, are often simply misdiagnosed as heart attacks or strokes while many of them may be due to aneurysms.

U.S. surgeons treat approximately 50,000 abdominal aortic aneurysms each year, typically by replacing the abnormal section of vessel with a plastic or fabric graft in an open surgical procedure. A less-invasive procedure that has more recently been used is the placement of a stent graft at the aneurysm site. Stent grafts are tubular devices that span the aneurysm site to provide support without replacing a section of the vessel. The stent graft, when placed within a vessel at an aneurysm site, acts as a barrier between blood flow and the weakened wall of a vessel, thereby decreasing pressure on the damaged portion of the vessel. This less invasive approach to treat aneurysms decreases the morbidity seen with conventional aneurysm repair. Additionally, patients whose multiple medical comorbidities make them excessively high risk for conventional aneurysm repair are candidates for stent grafting.

While stent grafts represent improvements over previously-used vessel treatment options, there are still risks associated with their use. The most common of these risks is migration of the stent graft due to hemodynamic forces within the vessel. Stent graft migrations can lead to endoleaks, a leaking of blood into the aneurysm sac between the outer surface of the graft and the inner lumen of the blood vessel which can increase the risk of vessel rupture. Such migrations of stent grafts are especially possible in curved portions of vessels where hemodynamic forces are asymmetrical placing uneven forces on the stent graft. Additionally, the asymmetrical hemodynamic forces can cause remodeling of an aneurysm sac which leads to increased risk of aneurysm rupture and increased endoleaks.

Based on the foregoing, one goal of treating aneurysms is to provide stent grafts that do not migrate. To achieve this goal, stent grafts with stainless steel anchoring barbs that engage the vessel wall have been developed. Additionally, endostaples that fix stent grafts more readily to the vessel wall have been developed. While these physical anchoring devices have proven to be effective in some patients, they have not sufficiently ameliorated stent graft migration associated with current treatment methods in all cases.

An additional way to reduce the risk of stent graft migration is to administer to the treatment site, either before, during or relatively soon after implantation, a cell growth promoting factor (also known as an endothelialization factor). This administration can be beneficial because, normally, the endothelial cells that make up the portion of the vessel to be treated are quiescent at the time of stent graft implantation and do not multiply. As a result, the stent graft rests against a quiescent endothelial cell layer. If endothelialization factors are administered immediately before, during or relatively soon after stent graft deployment and implantation, the normally quiescent endothelial cells lining the vessel wall, and in intimate contact with the stent graft, will be stimulated to proliferate. The same will occur with smooth muscle cells and fibroblasts found within the vessel wall. As these cells proliferate they can grow around the stent graft such that the device becomes physically attached to the vessel wall rather than merely resting against it. This endothelialization helps to prevent stent graft migration, although it is not successful in all circumstances. Silk-containing stent grafts comprising an endoluminal stent and a graft are described in WO 2004/060424, wherein the silk induces the in vivo adhesion of the stent graft to vessel walls. A graft material may be attached to appropriate portions of a wire as described in WO 99 43379. Therefore, there is still room for improvement in preventing stent graft migration.

### SUMMARY OF THE INVENTION

In view of the above, a stent graft according to present claim 1 is provided. Further aspects, features, details, and advantages of the present invention are apparent from the dependent claims, the specification, and the accompanying drawings.

The present invention provides methods and devices to assist in the fixation of stent grafts to vessel walls at treatment sites. Embodiments according to the present invention assist in the fixation of stent grafts by providing stent grafts and methods of making and using the same with one or more bare metal portions wherein one or more of the bare metal portions comprises a substance that can promote an inflammatory response. In one embodiment the inflammatory response is promoted near the ends of the stent graft. In alternative embodiments, the substance that promotes an inflammatory response is incorporated on portions of the stent graft that are not at or near the ends. An inflammatory response at various positions along the length of a stent graft can lead to the development of limited scar formation which can help to anchor the stent graft to the vessel wall thus contributing to the prevention of stent graft migration.

One embodiment according to the present invention is a stent graft comprising exposed bare metal portions and a substance on one or more of the bare metal portions wherein the substance promotes an inflammatory response. the substance is wrapped helically around one or more of the bare metal portions, the substance is a biocompatible and biodegradable polymer. selected from the group consisting of polyglycolic acid, poly~glycolic acid/poly-L-lactic acid copolymers, polycaprolactive, polyhydroxybutyrate/hydroxyvalerate copolymers, poly-L-lactide, polydioxanone, polycarbonates, and polyanhydrides. In another embodiment at least one of the bare metal portions is at the end of the stent graft.

In another embodiment according to the present invention, the substance is in the form of a bioresorbable suture. In another embodiment, the substance is in the form of a bioresorbable suture and is wrapped helically around one or more of the bare metal portions.

In a comparative example, the substance comprises cotton, silk and/or starch.

In another embodiment, the stent graft further comprises and releases an endothelialization factor. In another embodiment of the stent grafts according to the present invention, the endothelialization factor is selected from the group consisting of vascular endothelial growth factor (VEGF), platelet-derived growth factor (PDGF), plated-derived epidermal growth factor (PDEGF), fibroblast growth factors (FGFs), transforming growth factor-beta (TGF-β), platelet-derived angiogenesis growth factor (PDAF) and autologous platelet gel (APG) including platelet rich plasma (PRP), platelet poor plasma (PPP) and thrombin.

The present disclosure also exemplifies methods. One method comprises providing a stent graft comprising one or more exposed bare metal portions and a substance on one or more of the bare metal portions wherein the substance promotes an inflammatory response. In another example of the methods at least one of the provided bare metal portions is located at the end of the stent graft.

In other methods, the substance is in the form of a bioresorbable suture, the substance is wrapped helically around one or more of the bare metal portions, and/or, the substance is in the form of a bioresorbable suture and is wrapped helically around one or more of the bare metal portions.

In another example of the methods the substance is a biocompatible and biodegradable polymer. In another example of the methods, the biocompatible and biodegradable polymer is selected from the group consisting of polyglycolic acid, poly~glycolic acid/poly-L-lactic acid copolymers, polycaprolactive, polyhydroxybutyrate/hydroxyvalerate copolymers, poly-L-lactide, polydioxanone, polycarbonates, and polyanhydrides.

In a comparative example of the methods, the substance used comprises cotton, silk and/or starch.

In another example of the methods, the stent graft further comprises and releases an endothelialization factor. In another example, the endothelialization factor is selected from the group consisting of vascular endothelial growth factor (VEGF), platelet-derived growth factor (PDGF), plated-derived epidermal growth factor (PDEGF), fibroblast growth factors (FGFs), transforming growth factor-beta (TGF-β), platelet-derived angiogenesis growth factor (PDAF) and autologous platelet gel (APG) including platelet rich plasma (PRP), platelet poor plasma (PPP) and thrombin.

Another example of the methods comprises providing a stent graft comprising one or more exposed bare metal ends and a substance on one or both of the bare metal ends wherein the substance promotes an inflammatory response, is in the form of a bioresorbable suture that is wrapped helically around the one or both of the bare metal ends and wherein the substance is selected from the group consisting of polyglycolic acid, poly~glycolic acid/poly-L-lactic acid copolymers, polycaprolactive, polyhydroxybutyrate/hydroxyvalerate copolymers, poly-L-lactide, polydioxanone, polycarbonates, polyanhydrides cotton, silk and starch; and positioning the stent graft at a treatment site wherein the substance contributes to the fixation of the stent graft to the vessel wall at the treatment site. In one embodiment, the treatment site can be an aneurysm site.

### BRIEF DESCRIPTION OF THE FIGURES

FIG. 1 depicts a schematic diagram of a representative stent graft that can be used in accordance with the present invention deployed at a treatment site.

FIG. 2 depicts a distal end of an injection and delivery catheter that can be used in accordance with the present invention.

FIG. 3 depicts a close-up view of the distal portion of a representative stent graft.

### DEFINITION OF TERMS

Prior to setting forth embodiments according to the present invention, it may be helpful to an understanding thereof to set forth definitions of certain terms that will be used hereinafter. Unless otherwise explained, all technical and scientific terms used herein have the same meaning as commonly understood by one of ordinary skill in the art to which this invention belongs. The singular terms "a," "an," and "the" include plural referents unless context clearly indicates otherwise. Similarly, the word "or" is intended to include "and" unless the context clearly indicates otherwise. The term "comprises" means "includes."

Aortic aneurysm: As used herein "aortic aneurysm" shall include a weak section of an animal's aorta. As used herein, an "aortic aneurysm" includes, without limitation, abdominal and thoracic aneurysms.

Animal: As used herein "animal" shall include mammals, fish, reptiles and birds. Mammals include, but are not limited to, primates, including humans, dogs, cats, goats, sheep, rabbits, pigs, horses and cows.

Drug(s): As used herein "drug" shall include any bioactive compound or composition having a therapeutic effect in an animal. Exemplary, non limiting examples include small molecules, peptides, proteins, hormones, DNA or RNA fragments, genes, cells, genetically-modified cells, endothelialization factors, matrix metalloproteinase inhibitors and autologous platelet gel.

Stent graft: As used herein "stent graft" shall include a fabric (or fabric and metal composite, and/or derivations and combinations of these materials) tube that reinforces a weakened portion of a vessel (in one instance, an aneurysm).

Endoleak: As used herein "endoleak" refers to the presence of blood flow past the seal between the end of a stent graft and the vessel wall (Type I leak), and into the aneurysmal sac, when all such flow should be contained within the stent graft's lumen.

Migration: As used herein "migration" refers to displacement of a stent graft from its intended implantation site after implantation.

Placed or implanted stent graft: As used herein "placed stent graft" or "implanted stent graft" shall include a surgically placed or implanted stent graft, either by invasive or non-invasive techniques.

Endothelialization Factors: As used herein, "endothelialization factors" include any agent that can promote cell growth and includes, without limitation, vascular endothelial growth factor (VEGF), platelet-derived growth factor (PDGF), plated-derived epidermal growth factor (PDEGF), fibroblast growth factors (FGFs), transforming growth factor-beta (TGF-β), platelet-derived angiogenesis growth factor (PDAF) and autologous platelet gel (APG) including platelet rich plasma (PRP), platelet poor plasma (PPP) and thrombin.

### DETAILED DESCRIPTION

Embodiments according to the present invention include devices that are useful in reducing the risk of implantable stent graft migration. More specifically, devices that promote implantable stent graft attachment to blood vessel luminal walls are provided. One embodiment provides devices useful for minimizing post-implantation stent graft migration following deployment at an aneurysmal treatment site and is also useful in preventing or minimizing post-implantation endoleak following stent-graft deployment at an aneurysmal treatment site. In some examples, devices also can lead to aneurysm shrinkage by inducing saccular thrombus conversion to organized tissue and collagen deposition.

As discussed above, an aneurysm is a swelling, or expansion of a vessel lumen at a defined point and is generally associated with a vessel wall defect. Aneurysms are often multi-factorial asymptomatic vessel diseases that if left unchecked can result in spontaneous rupture, often with fatal consequences. One method to treat aneurysms involves a highly invasive surgical procedure where the affected vessel region is removed and replaced with a synthetic graft that is sutured in place. However, this procedure is extremely risky and generally only employed in otherwise healthy vigorous patients who can be expected to survive the associated surgical trauma. Elderly and feeble patients are not candidates for these aneurysmal surgeries, and, before the development of stent grafts, remained untreated and at continued risk for sudden death.

In contrast to the described invasive surgical procedures, stent grafts can be deployed with a cut down procedure or percutaneously using minimally invasive procedures. Essentially, a catheter having a stent graft compressed and fitted into the catheter's distal tip is advanced through an artery to the aneurysmal site. The stent graft is then deployed within the vessel lumen juxtaposed to the weakened vessel wall forming an inner liner that insulates the aneurysm from the body's hemodynamic forces thereby reducing the risk of rupture. The size and shape of the stent graft is matched to the treatment site's lumen diameter and aneurysm length. Moreover, branched grafts are commonly used to treat abdominal aortic aneurysms that are located near the iliac branch.

While stent grafts provide a number of benefits, stent graft migration can be problematic, and tissue in-growth and endothelialization around the stent graft have been proposed as methods to reduce this risk. Embodiments according to the present invention provide mechanisms to further stimulate tissue in-growth at one or more portions of a stent graft by providing a stent graft with one or more bare metal portions with a substance on the one or more bare metal portions that triggers an inflammatory response. Other embodiments according to the present invention provide mechanisms to further stimulate tissue in-growth around a stent graft by providing a substance that promotes an inflammatory response on all or a subset of all bare metal portions found on a particular stent graft at a location other than the ends. In other examples, in addition to being found on bare metal portions of a stent graft, the substance that promotes an inflammatory response can be attached or woven into the material that forms the stent graft itself. As will be understood by one of skill in the art, however, and in light of further description provided herein, including the substance on bare metal portions that can then be attached to the stent graft material can provide a more efficient manufacturing process than including the substance within the stent graft material itself.

The substance that promotes an inflammatory response in accordance with the present invention can do so by recruiting macrophages to the site. Macrophages are the primary immune cells responsible for the conversion of thrombus to organized tissue and the deposition of collagen. Thus, any substance that is effective to recruit macrophages can be used with embodiments according to the present invention. In one embodiment, an absorbable material such as, without limitation, polyglycolic acid/lactide copolymer (PGLA) can be used. This substance, among others, degrades by hydrolysis to initiate a mild foreign body response, thus actively recruiting macrophages to the area.

Endothelialization may also be stimulated by induced angiogenesis, resulting in formation of new capillaries in the interstitial space and surface endothelialization. This has led to modification of stent grafts with vascular endothelial growth factor (VEGF) and fibroblast growth factors 1 and 2 (FGF-1, FGF-2). The discussion of these factors is for exemplary purposes only, as those of skill in the art will recognize that numerous other growth factors have the potential to induce cell-specific endothelialization. Co-pending United States Patent Application No. 10/977,545, filed October 28, 2004 discloses injecting autologous platelet gel (APG) into the aneurysmal sac and/or between an implanted stent graft and the vessel wall to induce endothelialization of the stent graft to prevent stent graft migration and resulting endoleak. The development of genetically-engineered growth factors also is anticipated to yield more potent endothelial cell-specific growth factors. Additionally it may be possible to identify small molecule drugs that can induce endothelialization. Thus, the stent grafts according to the present invention can improve tissue in-growth through providing substances that promote inflammatory responses near the ends of the stent graft, or at any other point along the length of the stent graft, and in some embodiments further by providing and releasing an endothelialization factor at one or more ends or along the length of the stent graft.

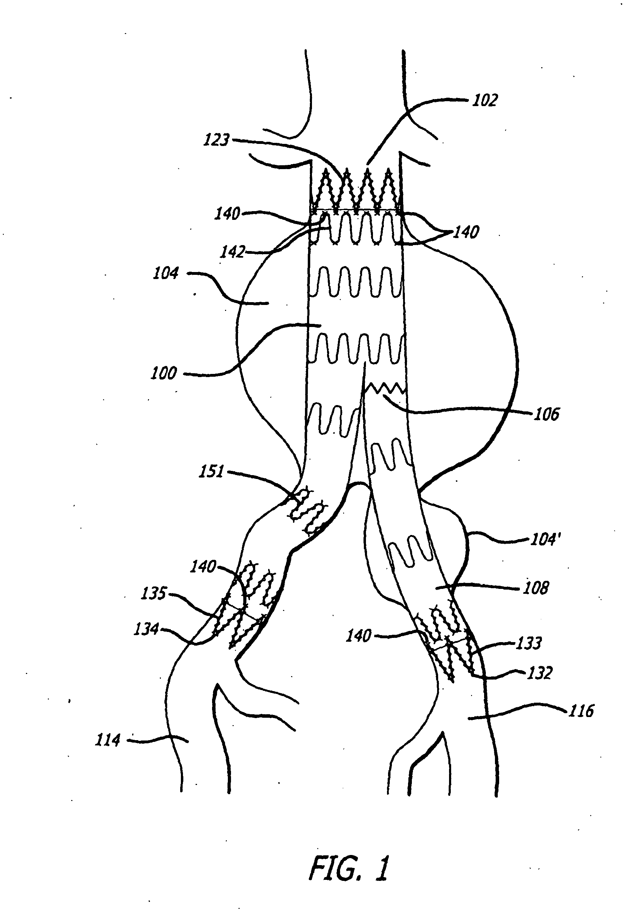

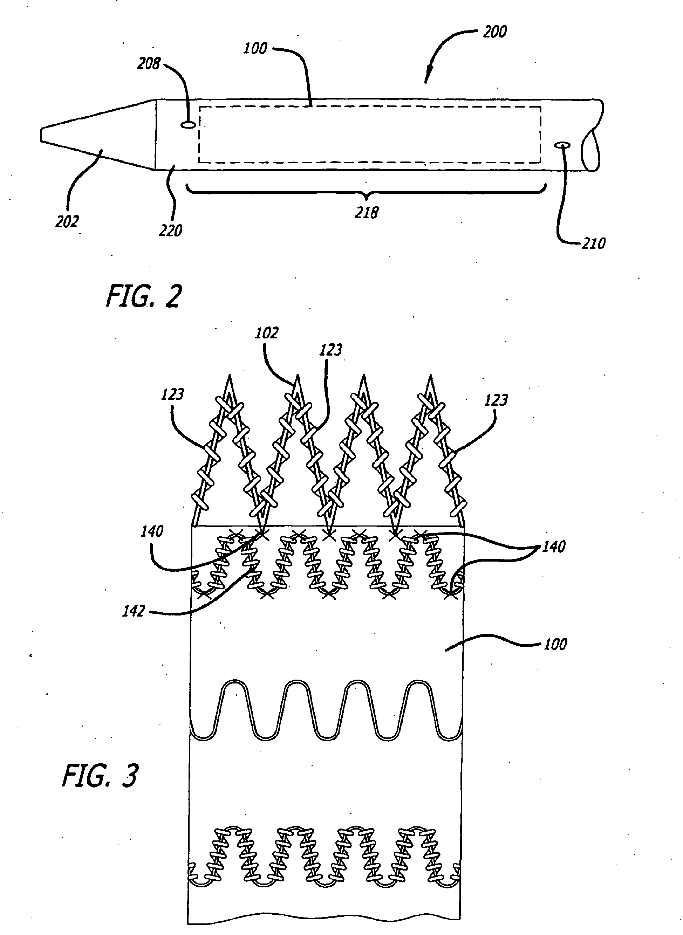

In one example, a stent graft is provided "pre-loaded" into a delivery catheter. In an exemplary stent graft deployment to the site of an abdominal aortic aneurysm, the stent graft **100** is fully deployed through the left iliac artery **114** to an aneurysm site **104** and **104'** (FIG. 1). The stent graft **100** depicted in FIG. 1 has a distal end **102** comprised of bare metal portion and an iliac leg **108** also with a bare metal portion **132** to anchor the stent graft in the iliac artery **116.** Stent graft **100** is deployed first in a first delivery catheter and the iliac leg **108** is deployed in a second delivery catheter. The stent graft **100** and iliac leg **108** are joined with a 2 cm overlap of the two segments **106.** In the embodiment depicted in FIG. 1, the bare metal portions **102, 132, 134** found at the ends of the stent graft comprise helically wrapped bioresorbable sutures **123, 133, 135.** These bare metal portions **102, 132, 134** are attached to the stent graft **100** at connection points **140** by any appropriate method including, without limitation, by stitching. Embodiments of the present invention can also comprise bare metal portions along the length of stent graft **100** such as those depicted by, for example, bare metal portions **142** and **151.** In one embodiment, bare metal portions, such as that depicted by **142,** can be provided solely for further structural support of stent graft **100** and do not include bioresorbable suture. As can be seen by bare metal portion **151,** these portions can also comprise segments of helically-wrapped bioresorbable suture. As will be understood by one of ordinary skill in the art, this bioresorbable suture can be found on any combination, number or position of bare metal portions on a particular stent graft. One embodiment of bare metal portions **102** and **142,** helically wrapped bioresorbable suture **123** and connection points **140** of stent graft **100** can be seen in more detail in FIG. 3.

As stated, for efficiency in manufacturing, the bioresorbable suture can be helically wrapped around bare metal portions before they are attached to the stent graft **100** by connection points **140** or other appropriate connection means. While not depicted in FIG. 1, this suture material can also be attached or stitched directly into the stent graft material between the bare metal portions of the stent graft. The helically wrapped bioresorbable sutures can be any material that can recruit macrophages to the area including a biocompatible and biodegradable polymer, such as polyglycolic acid, poly~glycolic acid/poly-L-lactic acid copolymers, polycaprolactive, polyhydroxybutyrate/hydroxyvalerate copolymers, poly-L-lactide, polydioxanone, polycarbonates, and polyanhydrides.

In another embodiment, a stent graft comprising a substance that promotes an inflammatory response on one or more bare metal portions is pre-loaded into a delivery catheter such as that depicted in FIG. 2. Stent graft **100** is radially compressed to fill the stent graft chamber **218** in the distal end **202** of delivery catheter **200.** The stent graft **100** is covered with a retractable sheath **220.** In one embodiment, catheter **200** has two injection (delivery) ports **208** and **210** (and associated lumens) for delivering drugs of choice to the treatment site. In these embodiments, drugs such as, without limitation, endothelialization factors can be injected through either or both of injection ports **208** and **210.** In another embodiment, one of the ports **208** or **210** can be an injection (delivery) port and the other can be an exit (evacuation or drain) port. In this embodiment, drugs can be introduced at the treatment site through one port and its associated lumen and displaced blood or other liquids at the treatment site can exit the area through the second port and associated lumen. This features is especially beneficial at aneurysm treatment sites where increased internal pressure at the treatment site can increase the risk of vessel rupture. Stent graft 100 is then deployed to the treatment site as depicted in FIG. 1.

The field of medical device coatings is well established and methods for coating stent grafts with drugs, with or without added polymers, are well known to those of skill in the art. Non-limiting examples of coating procedures include spraying, dipping, waterfall application, heat annealing, etc. The amount of coating applied to a stent graft can vary depending upon the desired effect of the compositions contained within the coating. The coating may be applied to the entire stent graft or to a portion of the stent graft.

Unless otherwise indicated, all numbers expressing quantities of ingredients, properties such as molecular weight, reaction conditions, and so forth used in the specification are to be understood as being modified in all instances by the term "about."

Variations on embodiments will become apparent to those of ordinary skill in the art upon reading the foregoing description.

In closing, it is to be understood that the embodiments according to the invention disclosed herein are illustrative. Other modifications may be employed. Thus, by way of example, but not of limitation, alternative configurations invention may be utilized in accordance with the teachings herein.

## Claims

1. A stent graft (100) comprising one or more exposed bare metal portions (102, 132, 134) and a substance (123, 133, 135) on one or more of said bare metal portions (102, 132, 134) wherein said substance (123, 133, 135) promotes an inflammatory response, wherein said substance (123, 133, 135) is wrapped helically around said one or more of said bare metal portions (102, 132, 134) and wherein said substance (123, 133, 135) is a biocompatible and biodegradable polymer selected from the group consisting of polyglycolic acid, poly∼glycolic acid/poly-L-lactic acid copolymers, polycaprolactive, polyhydroxybutyrate/hydroxyvalerate copolymers, poly-L-lactide, polydioxanone, polycarbonates, and polyanhydrides.

2. The stent graft according to claim 1, wherein at least one of said bare metal portions (102, 132, 134) is found at the end of said stent graft (100).

3. The stent graft according to claim 1 or 2, wherein said substance is in the form of a bioresorbable suture (123, 133, 135).

4. The stent graft according to any of the preceding claims, wherein said substance (123, 133, 135) is in the form of a bioresorbable suture.

5. The stent graft according to any of the preceding claims, wherein said stent graft (100) further comprises and releases an endothelialization factor selected from the group consisting of vascular endothelial growth factor (VEGF), platelet-derived growth factor (PDGF), plated-derived epidermal growth factor (PDEGF), fibroblast growth factors (FGFs), transforming growth factor-beta (TGF-β), platelet-derived angiogenesis growth factor (PDAF) and autologous platelet gel (APG) including platelet rich plasma (PRP), platelet poor plasma (PPP) and thrombin.

## Patentansprüche

1. Stent-Graft (100), umfassend ein oder mehrere exponierte, blanke Metallabschnitte (102, 132, 134) und eine Substanz (123, 133, 135) an einem oder mehreren der blanken Metallabschnitte (102, 132, 134), wobei die Substanz (123, 133, 135) eine Entzündungsreaktion fördert, wobei die Substanz (123, 133, 135) spiralförmig um den einen oder die mehreren der blanken Metallabschnitte (102, 132, 134) gewunden ist und wobei die Substanz (123, 133, 135) ein biologisch kompatibles und bioabbaubares Polymer ist, ausgewählt aus der Gruppe bestehend aus Polyglycolsäure, Polyglycolsäure/Poly-L-Milchsäure-Copolymeren, Polycaprolactiv, Polyhydroxybutyrat/Hydroxyvalerat-Copolymeren, Poly-L-Lactid, Polydioxanon, Polycarbonaten und Polyanhydriden.

2. Stent-Graft nach Anspruch 1, wobei sich mindestens einer der blanken Metallabschnitte (102, 132, 134) an dem Ende des Stent-Graft (100) befindet.

3. Stent-Graft nach Anspruch 1 oder 2, wobei die Substanz die Form einer biologisch resorbierbaren Naht (123, 133, 135) aufweist.

4. Stent-Graft nach einem der vorangehenden Ansprüche, wobei die Substanz (123, 133, 135) die Form einer biologisch resorbierbaren Naht aufweist.

5. Stent-Graft nach einem der vorangehenden Ansprüche, wobei das Stent-Graft (100) des Weiteren einen Endothelialisierungsfaktor umfasst und freisetzt, der ausgewählt ist aus der Gruppe bestehend aus vaskulärem endothelialen Wachstumsfaktor (VEGF), Plättchen-abgeleitetem Wachstumsfaktor (PDGF) Plättchen-abgeleitetem epidermalen Wachstumsfaktor (PDEGF), Fibroblast-Wachstumsfaktoren (FGFs), transformierendem Wachstumsfaktor-Beta (TGF-β), Plättchen-abgeleitetem Angiogenese-Wachstumsfaktor (PDAF) und autologem Plättchen-Gel (APG), einschließlich Plättchen-reichem Plasma (PRP), Plättchen-armem Plasma (PPP) und Thrombin.

## Revendications

1. Endoprothèse vasculaire (100) comprenant une ou plusieurs parties en métal nu exposées (102, 132, 134) et une substance (123, 133, 135) sur une ou plusieurs desdites parties en métal nu (102, 132, 134), ladite substance (123, 133, 135) promouvant une réponse inflammatoire, ladite substance (123, 133, 135) étant enroulée en hélice autour de l'une ou de plusieurs desdites parties en métal nu (102, 132, 134) et ladite substance (123, 133, 135) étant un polymère biocompatible et biodégradable choisi dans le groupe constitué par l'acide polyglycolique, les copolymères d'acide poly-glycolique/poly-L-lactique, le polycaprolactive, les copolymères de polyhydroxybutyrate/hydroxyvalérate, le poly-L-lactide, le polydioxanone, les polycarbonates, et les polyanhydrides.

2. Endoprothèse vasculaire selon la revendication 1, dans laquelle au moins l'une desdites parties en métal nu (102, 132, 134) se trouve à l'extrémité de ladite endoprothèse vasculaire (100).

3. Endoprothèse vasculaire selon la revendication 1 ou 2, dans laquelle ladite substance se trouve sous la forme d'une suture biorésorbable (123, 133, 135).

4. Endoprothèse vasculaire selon l'une quelconque des revendications précédentes, dans laquelle ladite substance (123, 133, 135) se trouve sous la forme d'une suture biorésorbable.

5. Endoprothèse vasculaire selon l'une quelconque des revendications précédentes, dans laquelle ladite endoprothèse vasculaire (100) comprend en outre et libère un facteur d'endothélialisation choisi dans le groupe constitué par le facteur de croissance endothélial vasculaire (VEGF), le facteur de croissance dérivé des plaquettes (PDGF), le facteur de croissance épidermique dérivé des plaquettes (PDEGF), les facteurs de croissance des fibroblastes (FGF), le facteur de croissance transformant bêta (TFG-β), le facteur de croissance angiogénique dérivé des plaquettes (PDAF), et le gel plaquettaire autologue (APG) y compris le plasma riche en plaquettes (PRP), le plasma pauvre en plaquettes (PPP) et la thrombine.
